# EUROPÄISCHE PATENTSCHRIFT

(11) **EP 0 972 006 B1**
(45) Veröffentlichungstag und Bekanntmachung des Hinweises auf die Patenterteilung: **03.09.2003**
(21) Anmeldenummer: 98912429.2
(22) Anmeldetag: 03.03.1998
(51) Int. Cl.: C11D 11/04, C11D 11/00, C11D 1/04, C11D 1/22

(54) **VERFAHREN ZUR HERSTELLUNG VON ANIONTENSIDGRANULATEN**
METHOD FOR PRODUCING ANIONIC SURFACTANT GRANULATES
PROCEDE DE PREPARATION DE GRANULES TENSIOACTIFS ANIONIQUES

(30) Priorität: 12.03.1997 DE 19710152
(43) Veröffentlichungstag der Anmeldung: 19.01.2000
(73) Patentinhaber: Cognis Deutschland GmbH & Co. KG, 40589 Düsseldorf (DE)
(72) Erfinder: LÜDER, Thomas, D-40764 Langenfeld (DE); SCHOLINAKIS, Konstantinos, D-40789 Monheim (DE); GUTSCHE, Bernhard, D-40724 Hilden (DE)
(86) Internationale Anmeldenummer: EP9801173
(87) Internationale Veröffentlichungsnummer: WO98040461

(56) Entgegenhaltungen:
- EP-A- 0 345 090
- EP-A- 0 402 112
- WO-A-96/06917

## Beschreibung

### Gebiet der Erfindung

Die Erfindung betrifft ein verbessertes Verfahren zur Herstellung von Aniontensidgranulaten, bei dem man wäßrige Aniontensidpasten in ihrer sauren Form bei definiertem Feststoffgehalt einer gleichzeitigen Neutralisation, Trocknung und Granulierung in einem Dünnschichtverdampfer bzw. - trockner unterwirft.

### Stand der Technik

Aniontenside werden üblicherweise in mehreren Verfahrensschritten hergestellt: auf die Sulfierung folgt die Neutralisation mit Laugen in wäßrigem Milieu, schließlich wird das Wasser in einem abschließenden Trocknungsvorgang wieder bis auf die gewünschte Menge entfernt. Die Herstellkosten werden dabei ganz wesentlich durch die Menge Wasser bestimmt, die schließlich aus dem Produkt entfernt werden muß. Es ist sofort klar, daß es ein Bestreben sein muß, diese Menge möglichst gering zu halten und den Schritt der Trocknung nach Möglichkeit ganz einzusparen.

Es wurden in der Vergangenheit daher eine Vielzahl von Anstrengungen unternommen, um solche Verfahren zur Verfügung zu stellen und die Verfahrensökonomie durch Verminderung der Energiekosten und Steigerung des Durchsatzes zu verbessern. Die Absenkung des Wassergehaltes in der Neutralisation führt infolge Gelbildung jedoch zu einem steilen Viskositätsanstieg der Produkte, der begleitet wird von Inhomogenitäten im Produkt aufgrund unzureichender Vermischung von saurem Aniontensid und Neutralisationsbase. Mit angepaßtem technischen Installationen lassen sich daher hochkonzentrierte Aniontensidpasten mit Restwassergehalten von mindestens 25 bis 30 Gew.-% gerade noch herstellen und trocknen.

Eine hiervon konzeptionell abweichend Vorgehensweise ist die Integration von Neutralisation und Trocknung in einem Verfahrensschritt. Aus der Deutschen Patentanmeldung **DE-A1 3741401** (Henkel) ist ein Verfahren bekannt, bei dem man wäßrige saure Aniontenside und wäßrige Neutralisationsbasen mit einem Gasstrom beaufschlagt und dann durch Versprühen trocknet. Gegenstand der internationalen Patentanmeldung **WO 96/06917** (Unilever) ist ein Verfahren zur Herstellung von Aniontensidgranulaten, bei dem man wäßrige, saure Aniontenside in einem horizontal angeordneten Dünnschichtverdampfer bzw. -trockner mit wäßrigen Neutralisationsmitteln vermischt, so daß neutralisierte Zubereitungen mit einem Wassergehalt oberhalb von 10, vorzugsweise oberhalb von 20 Gew.-% entstehen und diese dann bei Temperaturen oberhalb von 130°C bis auf den gewünschten Wassergehalt von weniger als 20 Gew.-%, vorzugsweise weniger als 15 Gew.-% abtrocknet und gleichzeitig granuliert. Der hohe Wassergehalt in der Neutralisation ist energetisch ungünstig und führt zudem zu einem vergleichsweise geringen Durchsatz. So ergibt sich bei Bilanzierung der dort beschriebenen Neutralisation von wäßrigem Alkylschwefelsäurehalbester mit 30 Gew.-%iger Natronlauge, daß dort zwischenzeitlich ein Produkt mit mindestens 25 Gew.-% Gesamtwassergehalt entsteht, welches dann entwässert wird.

Demgegenüber wird in der internationalen Patentanmeldung **WO 96/06916** (Unilever) ein Verfahren zur Trocknung von wäßrigen Aniontensidpasten in einem horizontalen Dünnschichtverdampfer vorgeschlagen, das bei leichtem Vakuum bis fast Normaldruck und Temperaturen oberhalb von 130°C arbeitet. Ein weiteres Merkmal dieses Verfahrens ist der Einsatz einer sehr hohen Umfanggeschwindigkeit der eingesetzten Rührorgane von mindestens 15 m/s, was einen direkten Wandkontakt praktisch ausschließt und zu hellfarbigen Produkten führt. Bei der Trocknung von wäßrigen Aniontensidpasten, insbesondere wäßrigen Pasten von Alkylsulfaten oder Alkylethersulfaten, besteht jedoch grundsätzlich die Gefahr unerwünschter Hydrolyse im Produkt. Schon kurzfristiges, punktuelles Absinken des pH-Wertes führt in Gegenwart von Wasser zur Rückspaltung, zur Bildung anorganischen Sulfats und zum Rückgang des Gehaltes an waschaktiver Substanz. Bei der Nacharbeitung der Lehre der WO 96/06916 hat die Anmelderin gefunden, daß sich über einen Zeitraum von mehreren Betriebsstunden ein hydrolysefreies Produkt nicht reproduzierbar herstellen läßt.

Demzufolge hat die Aufgabe der Erfindung darin bestanden, ein Verfahren zur Herstellung von Aniontensidgranulaten durch gleichzeitige Neutralisation, Trocknung und Granulation zur Verfügung zu stellen, das frei von den geschilderten Nachteilen ist.

### Beschreibung der Erfindung

Gegenstand der Erfindung ist ein Verfahren zur Herstellung von Aniontensidgranulaten durch gleichzeitige Neutralisation von sauren Aniontensiden mit Basen, Trocknung und Granulierung in einem horizontalen Dünnschichtverdampfer bzw. -trockner, welches sich dadurch auszeichnet, daß man die sauren Aniontenside und/oder die Basen als wäßrige Zubereitungen mit einem solchen Feststoffgehalt einsetzt, daß die Gesamtwassermenge in der Anlage zu keiner Zeit 15 Gew.-% übersteigt, und die Neutralisation und Trocknung im Gegenstrom mit Luft oder einen alkalisch eingestellten Gasstrom bei Temperaturen im Bereich von 120 bis 130°C durchführt.

Überraschenderweise wurde gefunden, daß sich die gleichzeitige Neutralisation und Trocknung im Dünnschichttrockner auch dann mit ausgezeichneten Ergebnissen durchführen läßt, wenn man den Apparat unmittelbar mit wasserfreien oder hochkonzentriert-wäßrigen, sauren Aniontensiden und hochkonzentrierten wäßrigen Basen beschickt, so daß während des gesamten Prozesses, also Neutralisation, Trocknung und Granulation der Gesamtwassergehalt 15 Gew.-%, vorzugsweise 10 Gew.-% nicht übersteigt. Gegenüber dem Verfahren der WO 96/06917 wurde die Gesamtwasserbilanz um bis zu 44 % reduziert und der Durchsatz um mehr als 60 % verbessert. Durch Teilrückführung von trockenem Produkt ist zudem eine weitere Steigerung des Durchsatzes möglich. Die Neutralisation/Trocknung wird im Bereich von 120 bis 130°C im Luft oder alkalisch eingestellten Gasstrom durchgeführt, was zu farblich einwandfreien, besonders rieselfähig Granulaten führt.

### Saure Aniontenside

Typische Beispiele für **anionische Tenside,** die im Sinne des erfindungsgemäßen Verfahrens hergestellt werden können, sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis), Alkyl(ether)phosphate sowie Sulfate von Ringöffnungsprodukten von Olefinepoxiden mit Wasser oder Alkoholen. Sofern die anionischen Tenside Polyglycoletherketten enthalten, können diese eine konventionelle, vorzugsweise jedoch eine eingeengte Homologenverteilung aufweisen. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J. Falbe (ed.), "Surfactants in Consumer Products", Springer Verlag, Berlin, 1987, S. 54-124** oder **J. Falbe (ed.), "Katalysatoren, Tenside und Mineralöladditive", Thieme Verlag, Stuttgart, 1978, S. 123-217** verwiesen. Die Tenside liegen vor der Trocknung in ihrer sauren, d.h. nicht neutralisierten Form vor und können entweder als wäßrige Zubereitungen mit einem Feststoffgehalt im Bereich von 80 bis 99,9, vorzugsweise 85 bis 95 Gew.-% oder aber wasserfrei eingesetzt werden. Vorzugsweise werden Carbonsäuren, Schwefelsäurehalbester von Alkoholen oder Alkoholethoxylaten sowie Alkylbenzol-sulfonsäuren eingesetzt, die nach der Neutralisation Seifen, Alkyl(ether)sulfate bzw. Alkylbenzol-sulfonate ergeben.

### Carbonsäuren

Carbonsäuren, die nach Neutralisation Seifen ergeben und als Einsatzstoffe für das erfindungsgemäße Verfahren in Betracht kommen, folgen der Formel **(I)**,

**R**^{**1**}**CO-OH (I)**

in der R¹CO für einen linearen oder verzweigten Alkyl- und/oder Alkenylrest mit 6 bis 22 Kohlenstoffatomen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure, Erucasäure, Guerbetsäuren sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure.

### Alkyl- und/oder Alkenylschwefelsäurehalbester

Alkyl- und/oder Alkenylschwefelsäurehalbestem, aus denen nach Neutralisation Alkyl- und/oder Alkenylsulfate entstehen, stellen die Sulfatierungsprodukte primärer Alkohole dar, die der Formel **(II)** folgen,

**R**^{**2**}**O-SO**_{**3**}**H (II)**

in der R² für einen linearen oder verzweigten, aliphatischen Alkyl- und/oder Alkenylrest mit 6 bis 22, vorzugsweise 12 bis 18 Kohlenstoffatomen steht. Typische Beispiele für Schwefelsäurehalbester, die Sinne der Erfindung als Rohstoffe eingesetzt werden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen'schen Oxosynthese erhalten werden. Weiterhin können auch Guerbetalkohole mit 16 bis 32 Kohlenstoffatomen als Rohstoffe dienen. Besonders bevorzugt sind Alkylschwefelsäurehalbester auf Basis von Laurylalkohol, Stearylalkohol sowie entsprechender Kokosfettalkohole.

### Schwefelsäurehalbester von Alkyl- und/oder Alkenylethern

Unter den Schwefelsäurehalbestem von Alkyl- und/oder Alkenylethem sind saure Aniontenside zu verstehen, die großtechnisch durch SO₃- oder Chlorsulfonsäure (CSA)-Sulfatierung von Oxoalkohol- bzw. Fettalkoholpolyglycolethern und nachfolgende Neutralisation hergestellt werden und dann Alkylund/oder Alkenylethersulfate ergeben. Im Sinne der Erfindung kommen als Ausgangsstoffe für die Trocknung Schwefelsäurehalbester in Betracht, die der Formel **(III)** folgen,

**R**^{**3**}**O-(CH**_{**2**}**CH**_{**2**}**O)**_{**m**}**SO**_{**3**}**H (III)**

in der R³ für einen linearen oder verzweigten Alkyl- und/oder Alkenylrest mit 6 bis 22 Kohlenstoffatomen und m für Zahlen von 1 bis 10 steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Weiterhin können auch Anlagerungsprodukte von Ethylenoxid an Guerbetalkohole mit 16 bis 32 Kohlenstoffatomen als Rohstoffe eingesetzt werden. Die Sulfatierungsprodukte können dabei sowohl eine konventionelle als auch eine eingeengte Homologenverteilung aufweisen. Besonders bevorzugt ist der Einsatz von Schwefelsäurehalbestem auf Basis von Addukten von durchschnittlich 2 bis 3 Mol Ethylenoxid an technische C_{12/14}- bzw. C_{12/18}- Kokosfettalkoholfraktionen.

### Alkylbenzolsulfonsäuren

Unter Alkylbenzolsulfonsäuren, die nach der Trocknung Alkylbenzolsulfonate ergeben, sind Sulfonierungsprodukte von Alkylbenzolen zu verstehen, die der Formel **(IV)** folgen,

**R**^{**4**}**-Ph-SO**_{**3**}**H (IV)**

in der R⁴ für einen Alkylrest mit 8 bis 18 Kohlenstoffatomen und Ph für einen Phenylrest steht. Typische Beispiele für Alkylbenzolsulfonsäuren sind Dodecyl- und Tetradecylbenzolsulfonsäure.

### Basen

Während man die sauren Aniontenside, sofern dies die Viskosität zuläßt, entweder wasserfrei oder nur mit geringen Menge Wasser versetzt, verwendet, werden die Neutralisationsmittel stets als hochkonzen-trierte wäßrige Zubereitungen verwendet. Hierunter sind Lösungen von Alkali- und/oder Erdalkalihydroxiden, -carbonaten und/oder -hydrogencarbonaten wie etwa Natriumhydroxid, Natriumcarbonat, Natriumhydrogencarbonat, Kaliumhydroxid, Kaliumcarbonat, Kaliumhydrogencarbonat oder Ammoniak zu verstehen, die einen nicht-wäßrigen Anteil von mindestens 35 aufweisen, der nach oben nur durch die maximale Löslichkeit begrenzt wird. Dementsprechend setzt man vorzugsweise etwa 50 bis 55 Gew.-%ige wäßrige Lösungen von Natriumhydroxid oder Natriumcarbonat ein.

### Neutralisieren, Trocknen und Granulieren in der dünnen Schicht

Die gleichzeitige Neutralisation, Trocknung und Granulierung erfolgt in einem horizontal angeordneten Dünnschichtverdampfer mit rotierenden Einbauten, wie er z.B. von der Firma VRV unter der Bezeichnung "Flash Dryer" oder der Firma Vomm unter der Bezeichnung "Turbodryer" vertrieben wird. Hierbei handelt es sich, vereinfacht dargestellt, um ein Rohr, das über mehrere Zonen hinweg unterschiedlich temperiert werden kann. Über eine oder mehrere Wellen, die mit Blättern oder Flugscharen als rotierende Einbauten versehen sind, wird die Mischung aus saurem Aniontensid und Neutralisationsbase, die über eine Pumpe eindosiert wird, gegen die beheizte Wandung geschleudert, an der die Trocknung in einer dünnen Schicht von typischerweise 1 bis 10 mm Stärke erfolgt. In einer besonderen Ausführungsform des Verfahrens werden das saure Aniontensid und die Neutralisationsbasen getrennt eindosiert, so daß die Vermischung im Dünnschichtverdampfer bzw. -trockner eintritt. Im Sinne der Erfindung hat es sich als vorteilhaft erwiesen, an den Dünnschichtapparat einen Temperaturgradienten von 120 bis 130°C (Produkteinlaß) auf 20°C (Produktaustrag) anzulegen. Hierzu können beispielsweise die beiden ersten Zonen des Verdampfers auf 120 bis 130°C geheizt und die letzte auf 20°C gekühlt werden. Höhere Trocknungstemperaturen sind zwar möglich, haben sich im Hinblick auf die thermische Labilität der Ein-satzstoffe als nicht vorteilhaft erwiesen. Im Hinblick auf die zur Hydrolyse neigenden Schwefelsäure-halbester ist eine möglichst niedrige Betriebstemperatur erwünscht. Der Dünnschichtverdampfer wird bei atmosphärischem Druck betrieben und im Gegenstrom mit Luft (Durchsatz 50 bis 150 m³/h) oder einem alkalisch eingestellten Gasstrom (z.B. Luft/Ammoniak = 1:1) begast. Die Eintrittstemperatur des Gases liegt in der Regel bei 20 bis 30, die Austrittstemperatur bei 90 bis 110°C.

Nach der Trocknung hat es sich weiterhin als sehr vorteilhaft erwiesen, die noch etwa 50 bis 70°C heißen Granulate auf ein Förderband, vorzugsweise eine Schwingrinne zu geben und dort rasch, d.h. innerhalb einer Verweilzeit von 20 bis 60 s, mit Umgebungsluft auf Temperaturen von etwa 30 bis 40°C abzukühlen. Zur weiteren Verbesserung der Beständigkeit gegenüber unerwünschter Wasseraufnahme kann man die Granulate besonders hydrolyseempfindlicher Tenside, wie beispielsweise Alkylsulfate, auch anschließend durch Zugabe von 0,5 bis 2 Gew.-% Kieselsäure abpudem.

### Gewerbliche Anwendbarkeit

Die nach der erfindungsgemäßen Verfahren erhältlichen Granulaten können entweder im Apparat nach der Neutralisationszone oder anschließend mit weiteren Inhaltsstoffen von pulverförmigen oberflächenaktiven Mitteln, wie beispielsweise Turmpulvem für Waschmittel abgemischt werden. Es ist ferner problemlos möglich, die Pulver in wäßrige Zubereitungen einzuarbeiten. Tatsächlich werden bei Verwendung der Pulver gegenüber den wäßrigen Ausgangs-pasten keine Unterschiede in den anwendungstechnischen Eigenschaften beobachtet. Auch in Syndet-seifenrezepturen oder Zahnpasten lassen sich die Granulate beispielsweise zusammen mit Fettsäuren, Fettsäuresalzen, Stärke, Polyglycol und dergleichen ohne weiteres einarbeiten.

### Beispiele

### Beispiel 1 (nicht erfindungsgemäß)

Die Herstellung der Granulate erfolgte in einem Flash Dryer der VRV S.p.A., Maitand/IT. Es handelte sich hierbei um einen horizontal angeordneten Dünnschichttrockner (Länge 1100 mm, Innendurchmesser : 155 mm) mit 4 Wellen und 22 Blättern, deren Abstand zur Wandung 2 mm betrug. Der Trockner besaß drei separate Heiz- bzw. Kühlzonen und eine Wärmeaustauscherfläche von insgesamt 0,44 m². Der Betrieb erfolgte bei Normaldruck. Über zwei Schwingpumpen wurden separat auf 50°C temperierte wasserfreie Dodecylbenzolsulfonsäure und 50 Gew.-%ige wäßrige Natriumhydroxidlösung mit einem Durchsatz von 11,5 kg/h in den Dünnschichttgrockner gepumpt. Die für die vollständige Durchmischung der spontanen Reaktion erforderliche Mikromischung der Komponenten wurde innerhalb des Flash Dryers durch den schnelldrehenden Rotor (Umfangsgeschwindigkeit 25 m/s) gewährleistet. Die Heizzonen 1 und 2 des Flash Dryers wurden auf 170°C und die Kühlzone 3 auf 20°C eingestellt. Der Flash Dryer wurde mit Luft (ca. 110 m³/h) begast; die Gasaustrittstemperatur betrug ca. 65°C. Das vorgetrocknete, noch etwa 60°C heiße Granulat wurde auf eine Schwingrinne (Länge 1 m) gegeben, mit Raumluft begast und innerhalb von 30 s auf etwa 40°C abgekühlt. Anschließend wurde es mit etwa 1 Gew.-% Kieselsäure (Sipernat® 50 S) abgepudert. Es wurde ein trockenes, rein-weißes, auch nach längerer Lagerung an der Luft rieselfähiges und nicht-klumpendes Granulat erhalten, das nach Einarbeitung in Waschmittelrezepturen keine Unterschiede zu einem durch Sprühtrocknung hergestellten Vergleichsprodukt zeigte. Die Kenndaten des Granulats sind in Tabelle 1 wiedergegeben.

### Beispiel 2 (nicht erfindungsgemäß)

Analog Beispiel 1 wurde eine ölsäurehaltige Talgfettsäure (Edenor® PK 1805) eingesetzt. Die Ergebnisse sind in Tabelle 1 zusammengefaßt.

**Tabelle 1**

| **Kenndaten des Flash Dryer Granulats** | | |
|---|---|---|
| **Kenndaten** | **1** | **2** |
| Waschaktivsubstanz [Gew.-%] | 95,8 | 95,6 |
| Unsulfiertes bzw. freie Fettsäure [Gew.-%] | 1,4 | 1,4 |
| Natriumhydroxid [Gew.-%] | 1,0 | 0,9 |
| Natriumsulfat [Gew.-%] | 1,6 | 1,7 |
| Wasser [Gew.-%] | 0,2 | 0,4 |
| Schüttdichte [g/l] | 566 | 577 |
| Kornspektrum [Gew.-%] | | |
| < 0,1 mm | 0 | 0 |
| > 0,1 mm | 0,1 | 0,1 |
| > 0,2 mm | 0,4 | 0,7 |
| > 0,4 mm | 8,9 | 9,1 |
| > 0,8 mm | 52,0 | 46,7 |
| > 1,6 mm | 37,5 | 42,1 |
| > 3,2 mm | 1,1 | 1,3 |

## Patentansprüche

1. Verfahren zur Herstellung von Aniontensidgranulaten durch gleichzeitige Neutralisation von sauren Aniontensiden mit Basen, Trocknung und Granulierung in einem horizontalen Dünnschichtverdampfer bzw. -trockner, **dadurch gekennzeichnet, daß** man die sauren Aniontenside und/oder die Basen als wäßrige Zubereitungen mit einem solchen Feststoffgehalt einsetzt, daß die Gesamtwassermenge in der Anlage zu keiner Zeit 15 Gew.-% übersteigt, und die Neutralisation und Trocknung im Gegenstrom mit Luft oder einen alkalisch eingestellten Gasstrom bei Temperaturen im Bereich von 120 bis 130°C durchführt.

2. Verfahren nach Anspruch 1, **dadurch gekennzeichnet, daß** man anionische Tenside in ihrer Säureform einsetzt, die ausgewählt sind aus der Gruppe, die gebildet wird von Carbonsäuren, Schwefelsäurehalbester von Alkoholen und/oder Alkoholethoxylaten sowie Alkylbenzolsulfonsäuren.

3. Verfahren nach den Ansprüchen 1 und 2, **dadurch gekennzeichnet, daß** man die sauren Aniontenside in wasserfreier Form einsetzt.

4. Verfahren nach den Ansprüchen 1 bis 3, **dadurch gekennzeichnet, daß** man als Basen wäßrige Lösungen von Alkali- und/oder Erdalkalihydroxiden, -carbonaten und/oder -hydrogencarbonaten oder Ammoniak mit einem nicht-wäßrigen Anteil von mindestens 35 Gew.-% einsetzt.

5. Verfahren nach den Ansprüchen 1 bis 4, **dadurch gekennzeichnet, daß** man das Verfahren bei Atmosphärendruck durchführt.

## Claims

1. A process for the production of anionic surfactant granules by simultaneous neutralization of acidic anionic surfactants with bases, drying and granulation in a horizontal thin-layer evaporator or dryer, **characterized in that** the acidic anionic surfactants and/or the bases are used in the form of water-containing preparations with such a solids content that the total quantity of water in the evaporator/dryer at no time exceeds 15% by weight and the neutralization and drying step is carried out in countercurrent with air or an alkaline gas stream at temperatures of 120 to 130°C.

2. A process as claimed in claim 1, **characterized in that** anionic surfactants in their acid form selected from the group consisting of carboxylic acids, sulfuric acid semiesters of alcohols and/or alcohol ethoxylates and alkyl benzenesulfonic acids are used.

3. A process as claimed in claims 1 and 2, **characterized in that** the acidic anionic surfactants are used in water-free form.

4. A process as claimed in claims 1 to 3, **characterized in that** aqueous solutions of alkali metal and/or alkaline earth metal hydroxides, carbonates and/or hydrogen carbonates or ammonia with a non-aqueous component of at least 35% by weight are used as the bases.

5. A process as claimed in claims 1 to 4, **characterized in that** it is carried out at atmospheric pressure.

## Revendications

1. Procédé de fabrication de granulés de tensioactifs anioniques par neutralisation simultanée de tensioactifs anioniques acides avec des bases, séchage et granulation dans un évaporateur ou un sécheur à couche mince, horizontal,
**caractérisé en ce que**
l'on utilise les tensioactifs anioniques acides et/ou les bases sous forme de préparations aqueuses avec un extrait sec tel que la quantité totale d'eau dans l'installation ne dépasse à aucun moment 15 % en poids et on effectue la neutralisation et le séchage à contre-courant avec de l'air ou un courant de gaz ajusté à un pH alcalin, à des températures se situant dans une plage de 120 à 130°C.

2. Procédé selon la revendication 1,
**caractérisé en ce que**
les tensioactifs anioniques sont introduits sous leur forme acide, et sont choisis dans le groupe constitué d'acides carboxyliques, demi-esters sulfuriques d'alcools et/ou éthoxylates d'alcools ainsi qu'acides alkylbenzènesulfoniques.

3. Procédé selon les revendications 1 et 2,
**caractérisé en ce que**
l'on utilise les tensioactifs anioniques acides sous forme anhydre.

4. Procédé selon les revendications 1 à 3,
**caractérisé en ce que**
comme bases on utilise des solutions aqueuses d'hydroxydes, carbonates et/ou hydrogénocarbonates alcalins et/ou alcalino-terreux ou l'ammoniaque avec une proportion non aqueuse d'au moins 35 % en poids.

5. Procédé selon les revendications 1 à 4,
**caractérisé en ce que**
le procédé est effectué à la pression atmosphérique.
